# EUROPEAN PATENT APPLICATION

(11) **EP 3 988 545 A1**
(43) Date of publication of application: **27.04.2022**
(21) Application number: 20826066.1
(22) Date of filing: 22.06.2020
(51) Int. Cl.: C07D 401/14

(54) **METHODS FOR PREPARING CDK4/6 INHIBITOR AND SALT AND INTERMEDIATE THEREOF**

(30) Priority: 21.06.2019 CN 201910540035
(71) Applicant: Gan & Lee Pharmaceuticals Co., Ltd., Beijing 101109 (CN)
(72) Inventor: ZHANG, Yining, Beijing 101109 (CN); YIN, Lei, Beijing 101109 (CN); YAO, Zhenglin, Beijing 101109 (CN)
(74) Representative: Ladendorf, Oliver
(86) International application number: PCT/CN2020/097378
(87) International publication number: WO 2020/253865

(57) **Abstract**

The present invention relates to methods for preparing 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine and a salt and an intermediate thereof. The methods provided herein have improved the existing synthesis methods, simplified the preparation process and increased the yield and purity, and can well meet the requirement of large-scale industrial manufacture.

## Description

### CROSS-REFERENCE TO RELATED APPLICATIONS

The present application claims priority to patent application No. 201910540035.7 entitled "METHODS FOR PREPARING CDK4/6 INHIBITOR AND SALT AND INTERMEDIATE THEREOF" and filed with China National Intellectual Property Administration on June 21, 2019, the content of which is incorporated herein by reference in its entirety.

### TECHNICAL FIELD

The present invention relates to: (1) a method for preparing a CDK4/6 inhibitor, wherein the CDK4/6 inhibitor is 5-fluoro-4-(7'-fluoro-2'-methylspiro[cyclopentane-1,3'-indol]-5'yl)-*N-*(5-(1-methylpiperidin-4-yl)pyridin-2-yl)pyrimidin-2-amine; (2) a method for preparing an intermediate of 5-fluoro-4-(7'-fluoro-2'-methylspiro [cyclopentane-1,3'-indol]-5'-yl)-*N*-(5-(1-methylpiperidin-4-yl)pyridin-2-yl)pyrimidin-2-amine; and (3) a method for preparing fumarate of 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine.

### BACKGROUND

The structure of 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine (a compound of formula II) is as follows:

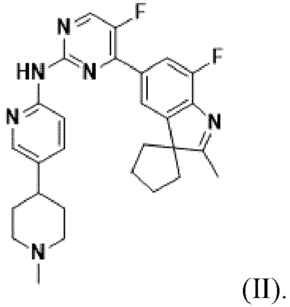

The synthesis method of this compound is described in Example 17 of patent application WO2017092635A1. WO2017092635A1 discloses that the compound is capable of selectively inhibiting cyclin dependent kinase CDK4/6 to allow cells to stop in G1 stage, and therefore the compound can be used for treating cell proliferative disorders and has a good application prospect in the pharmaceutical industry. However, for the original preparation method, the process is complex, column chromatography is required in each step of purification, the operation is complicated, the cost is high, and the yield is low, and thus the method cannot be applied to large-scale industrial manufacture. Therefore, it is highly desirable to provide a method for preparing the compound of formula (II) which can be applied to industrial manufacture.

### SUMMARY

The inventors have surprisingly obtained, through numerous experiments, novel methods for preparing a compound of formula (II) and an intermediate and a salt thereof. The methods are completely free of the purification by column chromatography, simplifies the preparation process, increases the yield and purity of products, and can well meet the requirement of large-scale industrial manufacture.

A first aspect of the present invention relates to a method for preparing a compound of formula (I). The compound is an intermediate for preparing 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine:

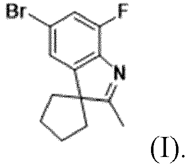

The preparation method comprises:
1) reacting (4-bromo-2-fluorophenyl)hydrazine or a salt thereof with 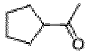 to 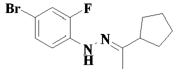 give a compound of formula (IV), and
2) forming the compound of formula 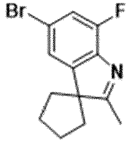 (I) using the compound of formula (IV), wherein the salt of (4-bromo-2-fluorophenyl)hydrazine is not particularly specified, and it includes, but is not limited to, 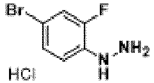 , sulfate of (4-bromo-2-fluorophenyl)hydrazine and *p*-toluenesulfonate of (4-bromo-2-fluorophenyl)hydrazine, but is preferably 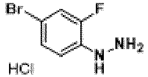 . Therefore, by first generating the hydrazone of formula (IV) that is easy to observe and detectable, the reaction becomes easier to control, the generation of impurities is reduced, and thus the step of purification by column chromatography can be omitted subsequently.

Optionally, the step 1) is performed under basic or neutral conditions. Preferably, the step 1) is performed in the presence of a base, which is not particularly specified as long as the reaction system of the step 1) can be made neutral or basic. The base can be an inorganic base or an organic base, preferably an organic base, more preferably diisopropylethylamine, triethylamine, pyridine, piperidine or *N,N-*dimethylaminopiperidine, still more preferably diisopropylethylamine or triethylamine, and most preferably triethylamine. The inventors have found that by performing the step 1) under basic or neutral conditions, in particular under basic conditions, or by making the reaction system of step 1) basic using a base, the intermediate hydrazone of formula (IV) can be produced first with a high conversion rate, and the production of impurities can be reduced.

Optionally, the step 2) is performed in the presence of an acid. Optionally, the acid is an organic acid, an inorganic acid or a Lewis acid, preferably sulfuric acid, hydrochloric acid, phosphoric acid, methanesulfonic acid, *p*-toluenesulfonic acid, acetic acid, ZnCl₂, FeCl₃, AlCl₃ or SnCl₄, and most preferably sulfuric acid.

Optionally, a solvent in the step 1) and the step 2) is an alcohol, preferably a C₁-C₅ linear or branched alkanol, more preferably methanol, ethanol, propanol or isopropanol, and still more preferably methanol or ethanol. The inventors have found that when the solvent is methanol or ethanol and H₂SO₄ is used as a catalyst in the step 2), the yield of the compound of formula (I) is high, impurities are easy to be removed, and the post-treatment is simple.

Optionally, the method further comprises: 3) extracting the compound of formula (I) obtained in the step 2) to give a crude product of the compound of formula (I). In this way, the compound of formula (I) can be isolated from the reaction solution with higher purity.

Optionally, the method further comprises: 4) purifying the crude product of the compound of the formula (I) obtained in the step 3) by slurrying. The method can meet the requirement of industrial manufacture by using slurrying instead of column chromatography for purification.

Optionally, a solvent used for the slurrying is a nonpolar solvent, and the nonpolar solvent is preferably *n*-heptane or *n*-hexane. The inventors have found that when a nonpolar solvent is used as the solvent for slurrying, a better purification effect can be achieved, such that the purity of the resulting compound of formula (I) is more than 98%, and the single impurity is less than 1%.

A second aspect of the present invention relates to a method for preparing a compound of formula (V). The compound of formula (V) is an intermediate for preparing 5-fluoro-4-(7'-fluoro-2'-methylspiro[cyclopentane-1,3'-indol]-5'-yl)-*N-*(5-(1-methylpiperidin-4-yl)pyridin-2-yl)pyrimidin-2-amine, and the preparation method comprises:
a) preparing a compound of formula (I) by the method described in the first aspect of the present invention; and
b) converting the compound of formula (I) to the compound of formula (V): 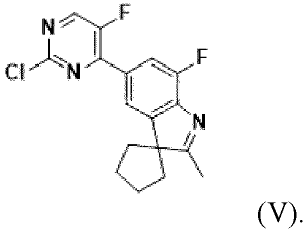 Optionally, the step b) comprises: (i) converting 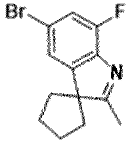 into 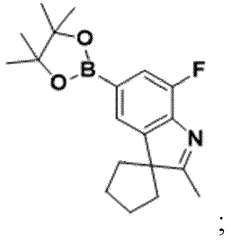 and (ii) converting the 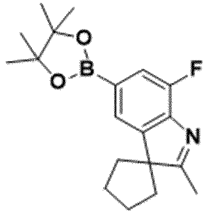 into the compound of formula

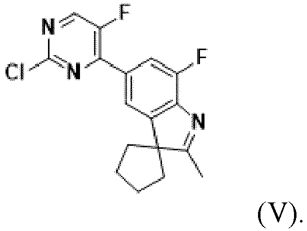

Optionally, the step (ii) further comprises crystallizing the compound of formula (V) after obtaining the compound of formula (V) by reaction. The method can meet the requirement of industrial manufacture as the process flow is simplified by using crystallization instead of column chromatography for purification.

Optionally, a solvent used for the crystallizing is an alcohol or a ketone; preferably, the alcohol is a C₁-C₅ linear or branched alkanol; more preferably, the C₁-C₅ linear or branched alkanol is methanol, ethanol, propanol or isopropanol, further preferably methanol or ethanol; preferably, the ketone is acetone. The inventors have found that when an alcohol or a ketone is used as the solvent for crystallizing, a better purification effect can be achieved, such that the purity of the resulting compound of formula (V) is more than 97%, and the individual impurity is less than 1%.

Optionally, there is also a step of treating the compound of formula (V) with activated carbon before crystallizing the compound of formula (V).

Optionally, the step (i) is performed in the presence of 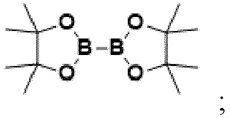 and/or the step (ii) is performed in the presence of 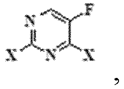 wherein X in the 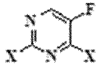 is chlorine, bromine or iodine, preferably chlorine.

Optionally, a reaction solvent of the step (i) is isopropanol or dioxane, preferably isopropanol; and/or a reaction solvent of the step (ii) is toluene or dioxane, preferably toluene.

A third aspect of the present invention relates to a method for preparing 2-amino-5-(1-methylpiperidin)pyridine of formula (VI), which comprises:
1) reacting 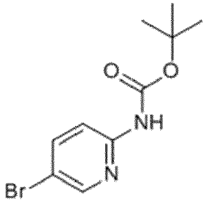 with *N*-methyl-4-piperidone to give 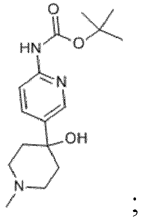
2) converting the 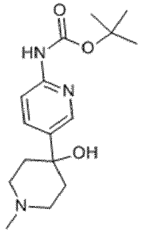 into 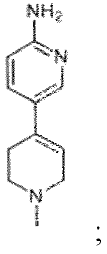 and
3) subjecting the 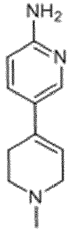 to hydrogenation reaction to give the compound of formula

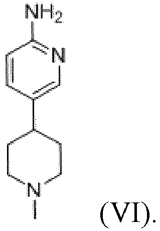

Compared with the synthesis method described in the Example 17 of the patent application WO2017092635A1, the synthesis method of the 2-amino-5-(1-methylpiperidin)pyridine of the present invention uses cheap *N*-methyl-4-piperidone instead of expensive boron reagent, so that the whole production cost can be reduced to about one tenth of the original cost, and meanwhile, the use of an expensive chiral palladium catalyst is avoided, and thus the production cost is further reduced, the risk of having residual heavy metal is lowered, and the method is more environment-friendly.

In one embodiment, the step 1) is performed in the presence of a Grignard reagent, an organolithium reagent or a combination thereof, wherein preferably, the Grignard reagent is selected from one or more of methylmagnesium chloride, ethylmagnesium chloride, isopropylmagnesium chloride, methylmagnesium bromide, ethylmagnesium bromide and isopropylmagnesium bromide, and more preferably the Grignard reagent is isopropylmagnesium chloride or isopropylmagnesium bromide; the organolithium reagent in the step 1) is one or more of *n*-butyllithium, *sec*-butyllithium, *tert*-butyllithium, lithium bis(trimethylsilyl)amide (LiHMDS) and lithium diisopropylamide (LDA), and more preferably, the organolithium reagent is selected from *n*-butyllithium, *sec*-butyllithium and lithium diisopropylamide (LDA).

In one embodiment, a crude product of the compound obtained in the step 1) is purified by slurrying. In a further embodiment, a solvent used for the slurrying is a combination of a nonpolar solvent and a polar solvent; preferably, the nonpolar solvent is *n*-heptane, and the polar solvent is methyl *tert*-butyl ether; preferably, the volume ratio of the *n*-heptane to the methyl tert-butyl ether is 1:1. The method can meet the requirement of industrial manufacture as the post-treatment is simple due to use of slurrying instead of column chromatography for purification.

In one embodiment, the step 2) is performed under acidic conditions, preferably in the presence of an acid; preferably, the acid is selected from one or more of *p-*toluenesulfonic acid, *p*-toluenesulfonic acid monohydrate, trifluoroacetic acid, methanesulfonic acid and hydrochloric acid, and more preferably, the acid is *p-*toluenesulfonic acid or *p*-toluenesulfonic acid monohydrate.

In one embodiment, the pressure of the hydrogenation reaction in the step 3) is 0.1-10 MPa, preferably 0.5-2 MPa, and more preferably 1 MPa. The inventors have surprisingly found, through numerous experiments, that the hydrogenation reaction in the process disclosed herein can be smoothly performed by controlling the pressure of hydrogenation reduction to be about 1 MPa, by-products are few, and the production safety is improved as the pressure is greatly reduced compared with the pressure for conventional catalytic hydrogenation reaction.

In one embodiment, the step 3) is performed in the presence of a hydrogenation catalyst, and the hydrogenation catalyst is not particularly specified as long as hydrogenation catalysis can be achieved; preferably the hydrogenation catalyst is selected from one or more of Pd/C, Pd(OH)₂, Raney nickel, platinum oxide and ammonium formate; preferably, the hydrogenation catalyst is Pd/C or Pd(OH)₂.

In one embodiment, a solvent used for hydrogenation reaction is one or more of C₁-C₅ linear and branched alkanols, preferably one or more of methanol, ethanol, propanol and isopropanol, and especially preferably isopropanol. Compared with the use of ethyl acetate/methanol described in patent application WO2017092635A1, the use of isopropanol as the solvent for hydrogenation reaction significantly increases the selectivity of the reduction reaction, reduces the generation of reduction by-products, improves the purity and quality of products, and enables the crude product of the compound of formula (VI) to be purified by slurrying. The method can meet the requirement of industrial manufacture by using slurrying instead of column chromatography for purification.

A fourth aspect of the present invention relates to a method for preparing a compound of formula (II), which comprises:
m) preparing a compound of formula (V) by the method described in the second aspect of the present invention; and
n) converting the compound of formula (V) into the compound of formula (II)

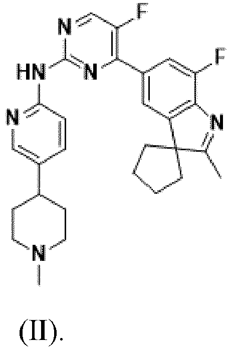

Optionally, the step n) is performed in the presence of 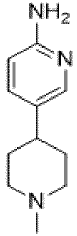 and a palladium catalyst. The palladium catalyst in the present invention refers to a catalyst containing element palladium (Pd), and exemplary examples of the palladium catalyst include, but are not limited to, Pd(dppf)Cl₂, Pd(OAc)₂, PdCl₂, Pd(PPh₃)₂Cl₂ and Pd₂(dba)₃. The palladium catalyst described herein is not particularly specified as long as it can convert the compound of formula (V) into the compound of formula (II), but preferably, the palladium catalyst is Pd(dppf)Cl₂, Pd(OAc)₂, PdCl₂, Pd(PPh₃)₂Cl₂ or Pd₂(dba)₃, and more preferably, the palladium catalyst is Pd(OAc)₂ or Pd₂(dba)₃.

Optionally, the compound of formula (VI) 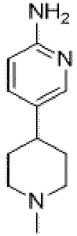 is prepared via the method described in the third aspect of the present invention.

Optionally, a solvent of the step n) is toluene, benzene or dioxane, preferably toluene.

Optionally, the step n) further comprises recrystallizing the compound of formula (II) to give a crude product of the compound of formula (II) after obtaining the compound of formula (II) by reaction. In this way, the method can meet the requirement of industrial manufacture as the process flow is simplified by using recrystallization instead of column chromatography for purification.

Optionally, solvents used for recrystallizing the compound of formula (II) comprise a first solvent and a second solvent, wherein the first solvent is toluene or dioxane, and the second solvent is a polar organic solvent; preferably, the second solvent is one or more of an alcohol, a ketone, a halogenated alkane, an ether and an ester; further preferably, the second solvent is one or more of a C₁-C₅ linear or branched alkanol, acetone, dichloromethane, chloroform, tetrahydrofuran and ethyl acetate; more preferably, the second solvent is one or more of methanol, dichloromethane, ethanol, propanol and isopropanol; most preferably, the second solvent is a combination of methanol and dichloromethane. The inventors have found that when recrystallization is performed by using the first and second solvents described herein, the crude product of the compound of formula (II) can be obtained with a higher purity. For example, in one embodiment of the present invention, the crude product of the compound of formula (II) can be obtained with a purity of 98.6% when the first solvent is toluene and the second solvent is a combination of methanol and dichloromethane.

Optionally, the step n) further comprises a step of removing palladium from the crude product of the compound of formula (II).

Optionally, the step of removing palladium is performed in the presence of a metal scavenger, wherein the metal scavenger of the present invention refers to a reagent capable of removing a substance containing a metal component from a target product, and examples of the metal scavenger include, but are not limited to, silica gel modified by various chemical groups. In one embodiment of the present invention, the metal scavenger is mercapto silica gel, that is, the step of removing palladium is performed in the presence of mercapto silica gel. In this way, the amount of residual palladium in the compound of formula (II) can meet the standard.

Optionally, the step n) further comprises a step of filtering resulting reaction solution and extracting resulting filtrate after the step of removing palladium. In the present invention, a solvent used for extracting the filtrate is not particularly specified as long as the compound of formula (II) disclosed herein can be dissolved, but preferably, the filtrate is extracted with a polar organic solvent; preferably, the polar organic solvent is one or more of an alcohol, a ketone, a halogenated alkane, an ether and an ester; further preferably, the polar organic solvent is one or more of a C₁-C₅ linear or branched alkanol, acetone, dichloromethane, chloroform, tetrahydrofuran and ethyl acetate; more preferably, the polar organic solvent is one or more of methanol, dichloromethane, ethanol, propanol and isopropanol; most preferably, the polar organic solvent is a combination of methanol and dichloromethane. In this way, the purity of the compound of formula (II) can be further increased, thereby making it possible to subsequently omit the step of purification by column chromatography.

Optionally, after the step of extracting is completed, there is a step of crystallizing from extract liquid to give the compound of formula (II) or a step of recrystallizing to give the compound of formula (II). A solvent used in the step of recrystallization is not particularly specified, but preferably the solvent used in the step of recrystallizing to give the compound of formula (II) is a combination of toluene and ethanol. In this way, the compound of formula (II) of the present invention can be obtained with a higher purity of more than 98% by means of crystallization or recrystallization. The method can meet the requirement of industrial manufacture as the process flow is simplified by using crystallization or recrystallization instead of column chromatography for purification.

A fifth aspect of the present invention relates to fumarate of 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine, which has a structure shown as formula (III) below:

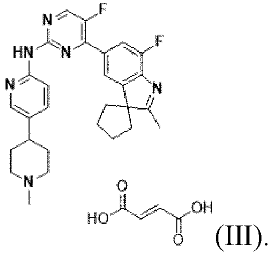

The fumarate according to the fifth aspect of the present invention has a higher crystallinity and a higher melting point, a higher solubility in water and in fasted state simulated intestinal fluid (FaSSIF) compared with the free base, and has a similar solubility in fasted state simulated intestinal fluid (FaSSIF) and fed state simulated intestinal fluid (FeSSIF), i.e., a lower risk of food effect; moreover, the fumarate unexpectedly has a low hygroscopicity comparable to the free base, and is stable in crystalline form after moisture absorption. Meanwhile, compared with free base, the fumarate has better physical and chemical stability under the conditions of high humidity and illumination.

A sixth aspect of the present invention relates to a method for preparing fumarate of 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine, which comprises reacting a compound of formula (II) with 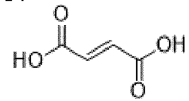 to give a compound of formula (III):

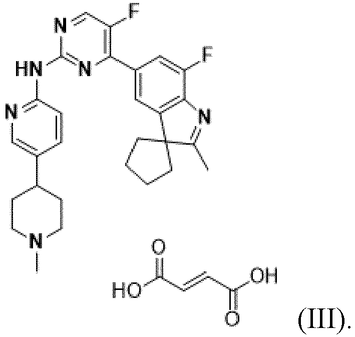

Preferably, the compound of formula (II) is prepared by the method described in the fourth aspect of the present invention.

Optionally, the reaction is performed in an organic solvent or an aqueous solution of the organic solvent; preferably, the organic solvent is one or more of an alcohol, a ketone, a halogenated alkane, an ether, an ester and an aromatic hydrocarbon; more preferably, the organic solvent is one or more of a C₁-C₅ linear or branched alkanol, acetone, ethyl acetate, dichloromethane, chloroform and tetrahydrofuran; further preferably, the organic solvent is one or more of methanol, ethanol, propanol, isopropanol, acetone, dichloromethane, chloroform, toluene and ethylbenzene; even more preferably, the reaction solvent is one of ethanol and dichloromethane, a mixture of ethanol and dichloromethane, one of ethanol and toluene, or a mixture of ethanol and toluene. Most preferably, the reaction solvent is a mixture of ethanol and dichloromethane, or a mixture of ethanol and toluene. This enables sufficient dissolution of the starting materials of reaction and acquisition of the compound of formula (III) in the desired crystalline form with high yield and purity.

The method further comprises crystallizing the compound of formula (III) after obtaining the compound of formula (III) by reaction. In this way, the compound of formula (III) can be obtained with higher purity by crystallization alone without column chromatography.

Optionally, the method further comprises: adding fumaric acid dropwise slowly to the 5-fluoro-4-(1'-fluoro-2'-methylspiro[cyclopentane-1,3'-indol]-5'-yl)-*N*-(5-(1-methylpiperidin-4-yl)pyridin-2-yl)pyrimidin-2-amine while mixing the 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine with the fumaric acid.

Optionally, the 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine is reacted with the fumaric acid at a temperature between 20 °C to a temperature of reaction solvent reflux.

Preferably, the method further comprises: dissolving the 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine before reacting the 5-fluoro-4-(7'-fluoro-2'-methylspiro [cyclopentane-1,3'-indol]-5'-yl)-*N*-(5-(1-methylpiperidin-4-yl)pyridin-2-yl)pyrimidin-2-amine with the fumaric acid.

Preferably, solvents used for dissolving 5-fluoro-4-(7'-fluoro-2'-methylspiro [cyclopentane-1,3'-indol]-5'-yl)-*N*-(5-(1-methylpiperidin-4-yl)pyridin-2-yl)pyrimidin-2-amine comprise a third solvent and a fourth solvent, wherein the third solvent is one or more of a ketone, an ether, an ester and an alcohol, preferably one or more alcohols, more preferably one or more of C₁-C₅ linear and branched alkanols, and especially preferably one or more of methanol, ethanol, propanol and isopropanol; the fourth solvent is toluene, dichloromethane, chloroform or any combination of two thereof; preferably, the fourth solvent is toluene, dichloromethane or a combination thereof; preferably, the fourth solvent is toluene.

The mixing volume ratio of the third solvent to the fourth solvent is not particularly specified as long as 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine can be dissolved. Preferably, the mixing volume ratio of the third solvent to the fourth solvent is 1:(0.1-10), preferably 1:(1-3), and most preferably 1:1.

Optionally, the method further comprises: distilling out the solvent used for dissolving the 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine before reacting the 5-fluoro-4-(1'-fluoro-2'-methylspiro[cyclopentane-1,3'-indol]-5'-yl)-*N*-(5-(1-methylpiperidin-4-yl)pyridin-2-yl)pyrimidin-2-amine with the fumaric acid, so as to ensure a more stable formation of the desired crystalline form of fumarate.

By adopting the preparation method described in the sixth aspect of the present invention, the desired crystalline form of fumarate can be repeatedly, stably and efficiently prepared, and the crystal quality is ensured.

### BRIEF DESCRIPTION OF THE DRAWINGS

FIG. 1 is HPLC chromatogram for compound 3.
FIG. 2 is HPLC chromatogram for compound 7, wherein PH-GLY-GLR2007-237FA-7 represents compound 7, and PH-GLY-GLR2007-237FA-5 represents compound 5.
FIG. 3 is ¹H-NMR graph of 2-Boc-amino-5-(4-hydroxy-1-methylpiperidin)pyridine (PH-GLY-GLR2007-237FA-8-1) in CDCl₃.
FIG. 4 is ¹H-NMR graph of 1'-methyl-1',2',3',6'-tetrahydro-[3,4'-bipyridin]-6-amine (PH-GLY-GLR2007-237FA-8-2) in CDCl₃.
FIG. 5 is ¹H-NMR graph of 2-amino-5-(1-methylpiperidin) pyridine (PH-GLY-GLR2007-237FA-8, compound 8) in CDCl₃.
FIG. 6 is HPLC chromatogram for a crude product of compound 9, wherein PH-GLY-GLR20087-237FA-9 represents the crude product of compound 9.
FIG. 7 is HPLC chromatogram for compound 9 in Example 4, wherein PH-GLY-GLR2007-237FA-9 represents compound 9.
FIG. 8 is ¹H-NMR graph of compound 9 in CDCl₃.
FIG. 9 is HPLC chromatogram for compound 9 in Example 5.
FIG. 10 is HPLC chromatogram for compound 9 in Example 6.
FIG. 11 is HPLC chromatogram for compound 10 in Example 7, wherein GLR2007-237FA represents compound 10.
FIG. 12 is ¹H-NMR graph of compound 10 in CD₃OD.
FIG. 13 is HPLC chromatogram for compound 10 in Example 8.
FIG. 14 is HPLC chromatogram for compound 10 in Example 9.

### DETAILED DESCRIPTION

The present invention is further illustrated by the following specific embodiments or examples. It should be noted that these embodiments or examples are not intended to limit the scope of the present invention.

In a specific embodiment of the present invention, illustratively shown are methods for preparing the compound of formula (I) (compound 3) of the first aspect of the present invention, the compound of formula (V) (compound 7) of the second aspect of the present invention, 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine (compound of formula (II) or compound 9) of the fourth aspect of the present invention, and the compound of formula (III) (compound 10) of the fifth aspect of the present invention, as shown in the process flowchart below.

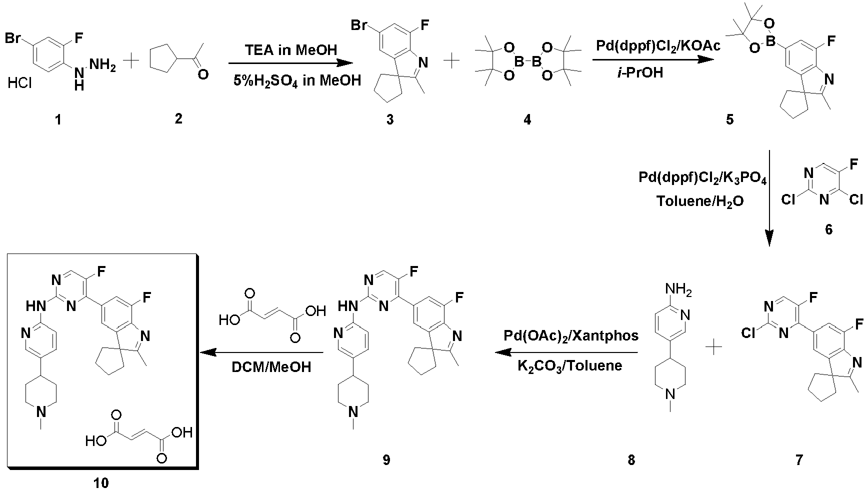

Regarding the method for preparing the compound of formula (I) (compound 3) of the first aspect of the present invention, the above process flow illustratively shows the synthesis route of the compound 3, that is, the compound 1 and the compound 2 are used as starting materials to generate hydrazone of the formula (IV) of the present invention first under basic conditions (in the presence of triethylamine), and then the hydrazone of formula (IV) is reacted in a solvent of 5% H₂SO₄ in methanol to give the compound 3.

Regarding the method for producing the compound of formula (V) (compound 7) of the second aspect of the present invention, the above process flow illustratively shows the synthesis route of the compound 7, that is, the compound 3 is used as a starting material and subjected to coupling reaction catalyzed by a palladium catalyst to give the compound 7.

Regarding the method for preparing 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine (compound of formula (II) or compound 9), the above process flow illustratively shows the synthesis route of the compound 9, that is, the compound 7 is used as a starting material and coupled with compound 8 in the presence of a catalyst to give the compound 9.

Regarding the method for preparing the compound of formula (III) (compound 10) of the sixth aspect of the present invention, the above process flow illustratively shows the synthesis route of the compound 10, that is, the compound 9 is used as a starting material and is reacted with fumaric acid to give the compound 10.

Abbreviations or definitions:
C₁-C₅ linear or branched alkanol: a linear or branched alkanol having 1 to 5 carbon atoms.
Slurrying: stirring and washing the target product with a solvent with poor solubility to the product to remove impurities attached to the surface of the product, wherein the temperature can be raised for stirring and then be lowered for filtering.
"Pd(dppf)Cl₂": [1,1'-bis(diphenylphosphino)ferrocene]palladium dichloride
Pd(OAc)₂: palladium acetate
Pd(PPh₃)₂Cl₂: bis(triphenylphosphine)palladium (II) chloride
Pd₂(dba)₃: tris(dibenzylideneacetone)dipalladium

### Example 1: Synthesis of Compound 3

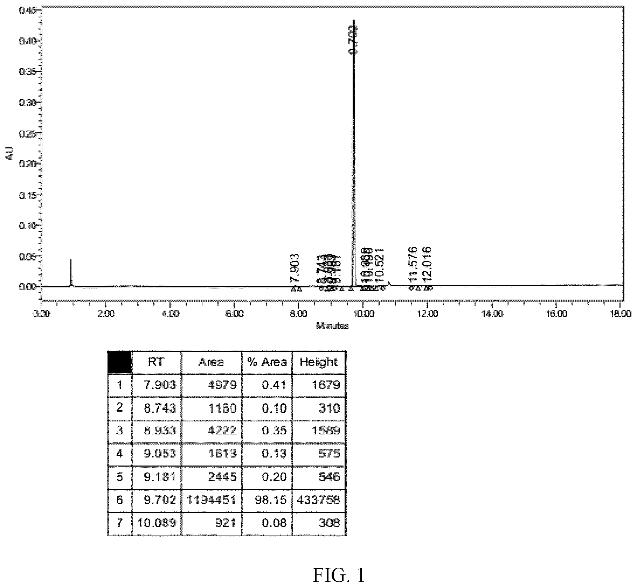

Methanol (5.0 vol.) and triethylamine (1.05 eq.) were added to a reaction kettle under nitrogen atmosphere, and (4-bromo-2-fluorophenyl)hydrazine hydrochloride (10 Kg, 1.0 eq.) was added with mechanical stirring. Then, cyclopentylethanone (4.628 Kg, 1.0 eq.) was added to the reaction kettle, and the reaction system was stirred at 30±10 °C until the reaction was completed. The reaction system was cooled, concentrated sulfuric acid (16.198 Kg, 3.2 eq.) was added dropwise slowly into the reaction system, and the temperature was maintained at ≤ 5 °C in the process of dropwise addition until it was completed. The reaction system was heated to 40-50 °C and stirred until the amount of the intermediate (hydrazone) was ≤ 1% as detected by HPLC. The reaction system was cooled to -10±2 °C. *n*-heptane (50 L, 5 vol.) and purified water (100 L, 10 vol.) were added into the reaction system, and then ammonia water was slowly added while stirring to adjust the pH value to 8-9. The resulting reaction system was stirred for 10 min and then subjected to liquid separation. Water (50 L, 5 vol.) was added to the organic phase, and the mixture was stirred for 10 min and then left to stand for liquid separation, and the aqueous phase was discarded. The organic phase was washed once with 2 mol/L hydrochloric acid (1 vol.) and extracted with 2 mol/L hydrochloric acid (5 vol. × 3). All aqueous phases with a 2 mol/L hydrochloric acid concentration were combined, and *n*-heptane (50 L, 5 vol.) was added. The reaction system was cooled to 0±5 °C, and ammonia water was added to adjust the pH value to 8-9. The resulting reaction system was stirred for 20 min and then left to stand for liquid separation. The organic phase was concentrated under reduced pressure at 40 °C, and a lot of solids were precipitated out. *n*-heptane (3 L, 0.3 vol.) was added, and the mixture was heated to 75-80 °C until the solution was clarified, and then stirred for 30 min. The solution was naturally cooled to room temperature to precipitate out crystals, and then the mixture was filtered and the filter cake was washed with *n*-heptane. The filter cake was dried under vacuum to give the compound 3 (4.2 Kg, 31% yield). A sample was taken for HPLC analysis. As shown in FIG. 1, the purity of the compound of formula I is 98.15%, and the maximum single impurity is 0.41%.

### Example 2: Synthesis of Compound 7

Isopropanol (10.0 vol.), the compound 3 obtained in Example 1 (5.0 Kg, 1.0 eq.), potassium acetate (2.10 Kg, 1.20 eq.) and bis(pinacolato)diboron (4.98 Kg, 1.1 eq.) were added to a reaction kettle under nitrogen atmosphere. The reaction system was stirred, and [1,1'-bis(diphenylphosphino)ferrocene]palladium dichloride (0.195 Kg, 0.015 eq.) was added. The reaction system was heated to 85±5 °C and maintained at this temperature until the reaction was completed. The reaction system was concentrated under reduced pressure and then extracted with purified water and toluene. Liquid separation was then performed, and the aqueous phase was discarded. The resulting organic phase was filtered and concentrated under reduced pressure to give compound 5.

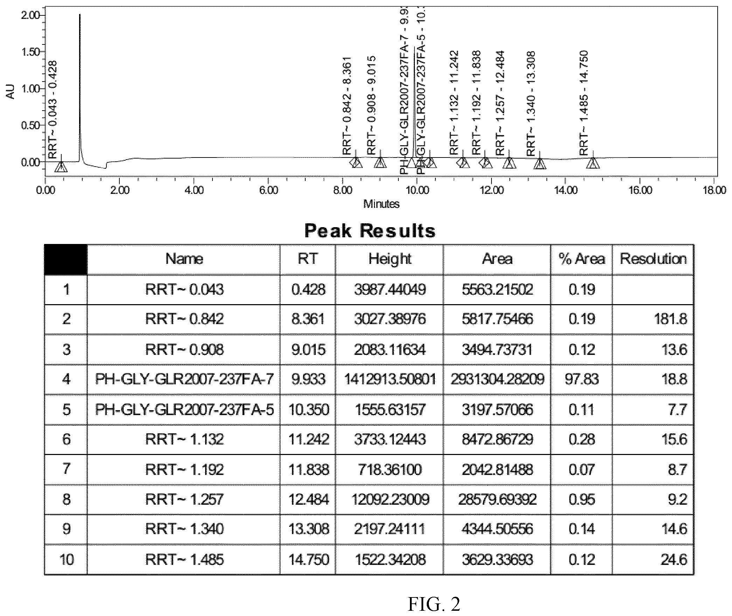

The compound 5 and toluene (8.0 vol.) were transferred into a reaction kettle and stirred. 2,4-dichloro-5-fluoropyrimidine (2.95 Kg, 1.0 eq.) and potassium phosphate (7.50 Kg, 2.0 eq.) were added, and the reaction system was stirred. [1,1'-bis(diphenylphosphino)ferrocene]palladium dichloride (0.195 Kg, 0.015 eq.) and purified water (1.0 vol.) were added to the reaction kettle successively. The resulting reaction system was heated to 85±5 °C and maintained at this temperature until the reaction was completed. The reaction system was cooled to 20-30 °C and centrifuged. The filter cake was washed twice with toluene and then discarded. The filtrate was washed with purified water and subjected to liquid separation, and the aqueous phase was discarded. The organic phase was transferred to a reaction kettle, and then activated carbon (10% w/w, 0.50 Kg) was added. The reaction system was stirred for about 2 h, cooled to 20-30 °C and subjected to suction filtration through celite (0.15 wt, 0.75 Kg) on a Buchner funnel. The filter cake was washed with toluene and the filtrate was collected, and the obtained organic phase was concentrated under reduced pressure. Methanol (5.0 vol.) was added, and the mixture was concentrated under reduced pressure to 2.0-3.0 volumes and then subjected to suction filtration. The filter cake was washed with methanol and then collected. A sample of the filter cake was taken for HPLC analysis. As shown in FIG. 2, the purity of the compound 7 is 97.83%, the content of the compound 5 is 0.11%, and the maximum single impurity is 0.95%. The production of the compound 7 was 4.175 Kg and its yield was 70.3%.

### Example 3: Synthesis of 2-amino-5-(1-methylpiperidin)pyridine (Compound 8)

In a specific embodiment of the present invention, a method for preparing 2-amino-5-(1-methylpiperidin)pyridine (compound 8) of the third aspect of the present invention is illustratively shown, as shown in the process flowchart below.

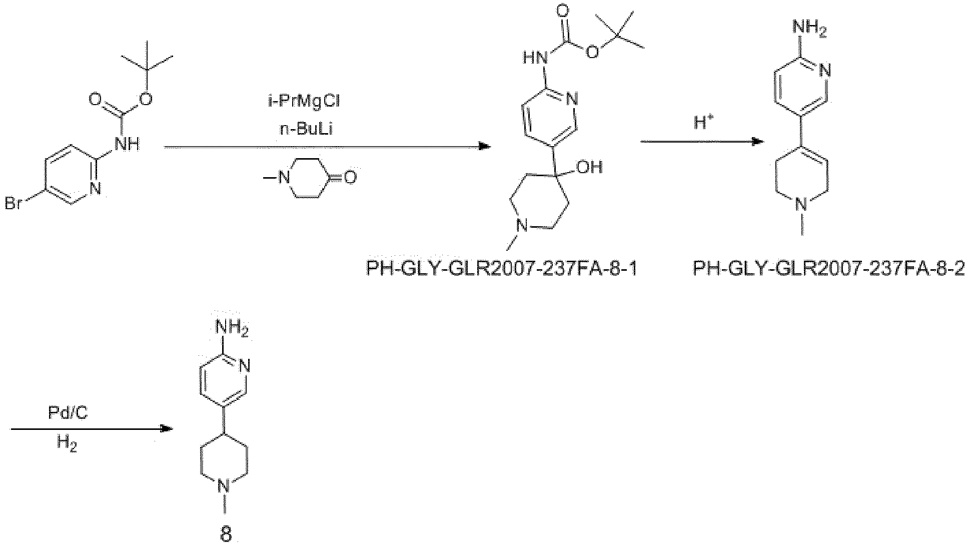

### 3.1. Synthesis of 2-Boc-amino-5-(4-hydroxy-1-methylpiperidin)pyridine (also known as PH-GLY-GLR2007-237FA-8-1)

THF (20 vol.) and 2-Boc-amino-5-bromopyridine (1.0 eq.) were sequentially added to a four-neck reaction flask, and then the reaction system was cooled to -5-0 °C, and isopropylmagnesium chloride (1.0 eq.) was added dropwise slowly. After the dropwise addition was completed, the resulting reaction system was stirred for 10 min at -5-0 °C. The reaction system was then cooled to -20 to -10 °C, and *n-*butyllithium (2.0 eq.) was added dropwise slowly. After the dropwise addition was completed, the reaction system was stirred for 30 min at -20 to -10 °C. The temperature was maintained at -20 to -10 °C, and a solution of *N*-methyl-4-piperidone in tetrahydrofuran (2.2 eq., 5 vol.) was added dropwise slowly. After the dropwise addition was completed, the reaction system was stirred for about 30 min at -20 to -10 °C. A sample was taken, water was added to quench the reaction, and the reaction was substantially completed as monitored by LC. The reaction system was heated to -5-0 °C, and water (10 vol.) was added dropwise slowly to the reaction flask. Liquid separation was performed, and the organic phase was collected and concentrated under reduced pressure. The resulting solid was slurried for 3 h at 20-30 °C with *n*-heptane and methyl tert-butyl ether (1:1, 10 vol.). Suction filtration was performed, and the filter cake was rinsed with *n*-heptane (2 vol.) and then collected. The filter cake was dried in a vacuum drying oven (50 °C, -0.1 MPa) for 16 h to give a white solid (57% yield).

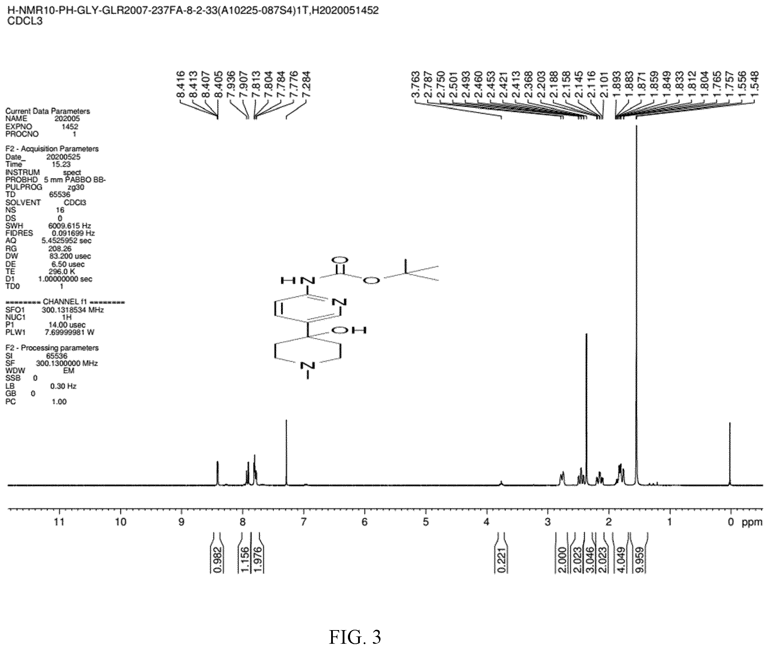

¹H-NMR (300 MHz, CDCl₃) δ8.41 (s, 1H), 7.92-7.78 (m, 3H), 2.78 (s, 1H), 2.44 (m, 2H), 2.37 (s, 3H), 2.14 (m, 2H), 1.81 (m, 4H), 1.55 (s, 9H), as shown in FIG. 3; MS (ESI) m/z = 308.2[M+H]⁺.

### 3.2. Synthesis of 1'-methyl-1',2',3',6'-tetrahydro-[3,4'-bipyridin]-6-amine (also known as PH-GLY-GLR2007-237FA-8-2)

Toluene (10 vol.), 2-Boc-amino-5-(4-hydroxy-1-methylpiperidin)pyridine (1.0 eq.) and *p*-toluenesulfonic acid monohydrate (4.0 eq.) were sequentially added to a four-neck reaction flask, and the reaction system was heated to 100-110 °C and maintained at this temperature for 20 h. A sample was taken, and the reaction was substantially completed as monitored by LC. Water was added slowly to the reaction flask, and then the reaction system was cooled to 20-30 °C. Liquid separation was performed, and the aqueous phase was collected. The pH of the aqueous phase was adjusted to 9-11 with 50% NaOH aqueous solution. Solids were precipitated out, and the mixture was stirred at 20-30 °C for 1 h. Suction filtration was performed, and the filter cake was collected. The filter cake was dried in a vacuum drying oven (50 °C, -0.1 MPa) for 16 h to give a white solid (72.4% yield).

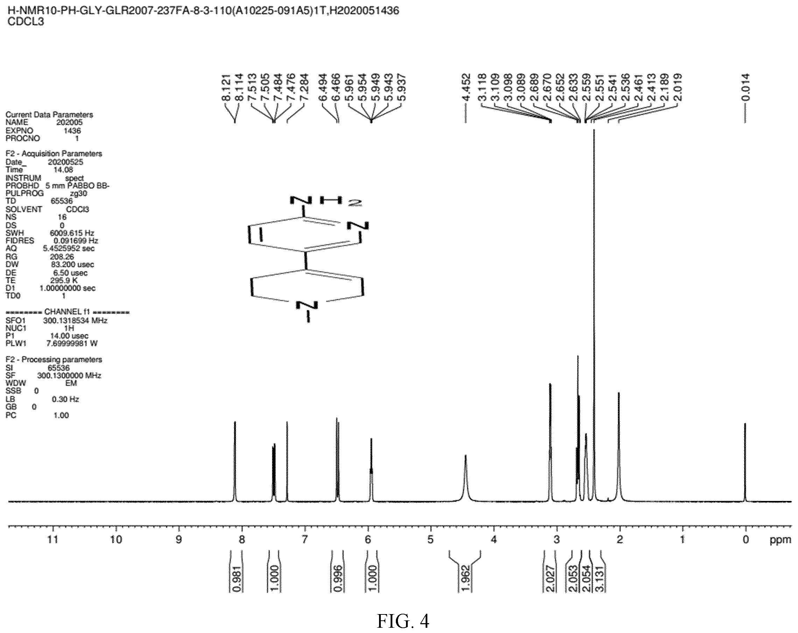

¹H-NMR (300 MHz, CDCl₃) δ8.11 (d, 1H), 7.54 (dd, 1H), 6.48 (d, 1H), 5.95 (m, 1H), 4.45 (s, 2H), 3.11 (m, 2H), 2.66 (m, 2H), 2.56 (m, 2H), 2.43 (s, 3H), as shown in FIG. 4; MS (ESI) m/z = 190.1[M+H]⁺.

### 3.3. Synthesis of 2-amino-5-(1-methylpiperidin)pyridine (also known as PH-GLY-GLR2007-237FA-8 or compound 8)

Isopropanol (10 vol.), PH-GLY-GLR2007-237FA-8-2 (1.0 eq.) and wet Pd/C (10 wt%) were added sequentially to an autoclave. The reaction system was heated to 70-80 °C and maintained at this temperature for about 18 h under hydrogen atmosphere (1 MPa). A sample was taken, and the reaction was substantially completed as monitored by LC. The reaction system was subjected to suction filtration through celite. The filter cake was rinsed with isopropanol (2 vol.), and the filtrate was collected. The filtrate was concentrated to 1-2 volumes, subjected to replacement with ethyl acetate (2 vol.) three times, and concentrated to 1-2 volumes. *n*-heptane (10 vol.) was added, and the mixture was stirred at 20-30 °C for 3 h. Suction filtration was performed, and the filter cake was collected. The filter cake was dried in a vacuum drying oven (50 °C, -0.1 MPa) for 16 h to give a white solid (94% yield).

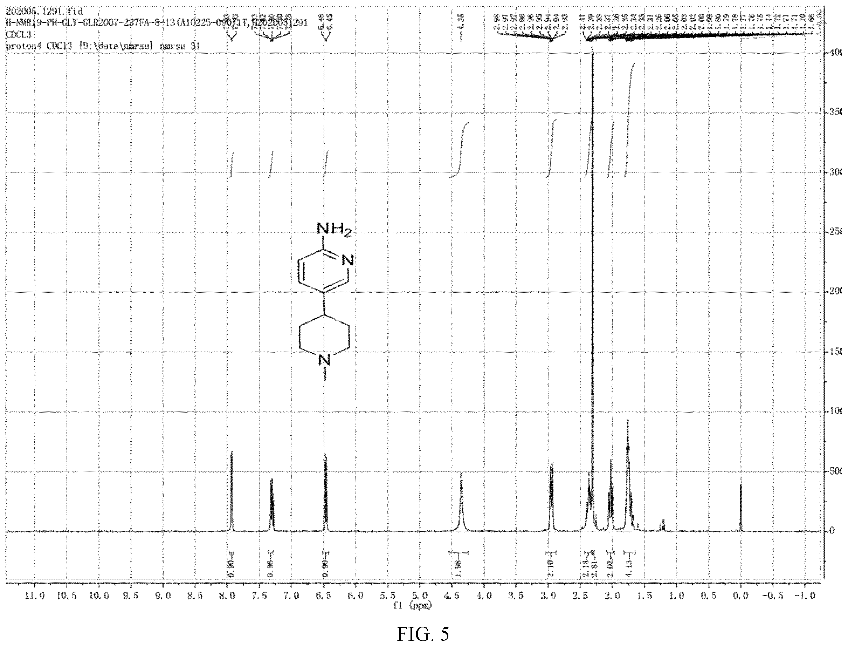

¹H-NMR (300 MHz, CDCl₃) δ7.93 (d, 1H), 7.30 (dd, 1H), 6.47 (d, 1H), 4.35 (s, 2H), 2.93 (m, 2H), 2.41 (m, 1H), 2.38 (s, 3H), 2.06 (m, 2H), 1.76 (m, 4H), as shown in FIG. 5; MS (ESI) m/z = 192.2[M+H]⁺.

### Example 4: Synthesis of 5-fluoro-4-(7'-fluoro-2'-methylspiro[cyclopentane-1,3'-indol]-5'-yl)-N-(5-(1-methylpiperidin-4-yl)pyridin-2-yl)pyrimidin-2-amine (Compound of Formula (II) or Compound 9)

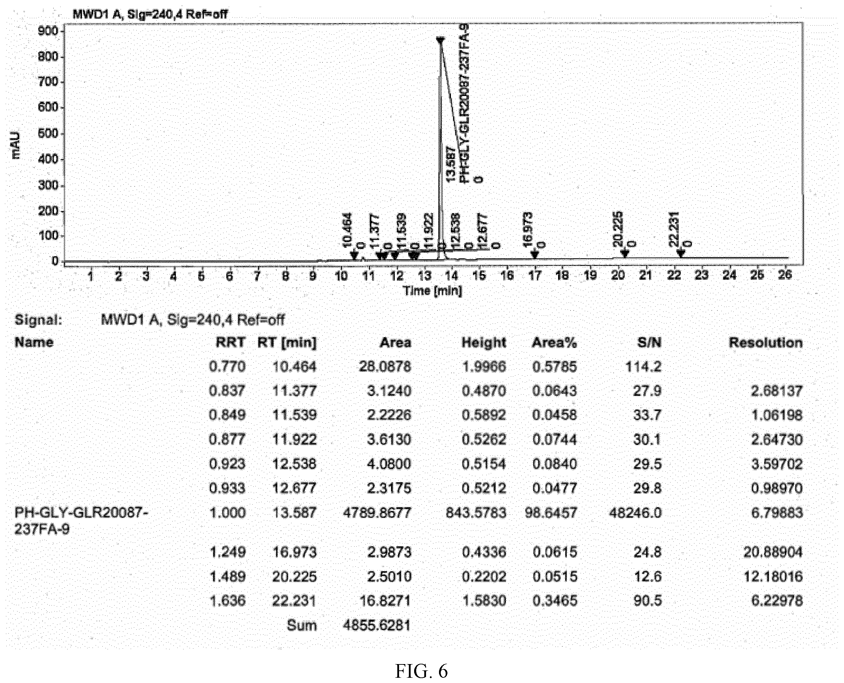

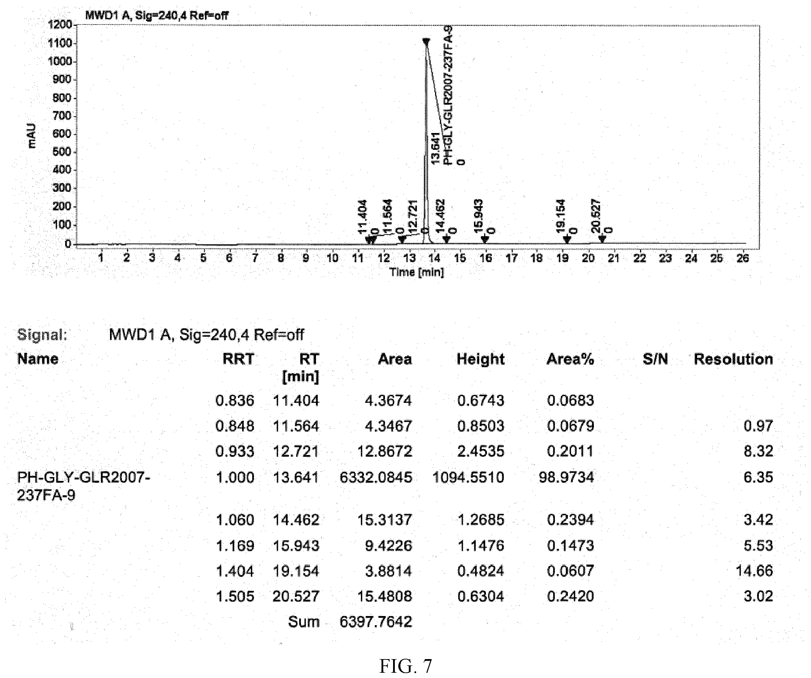

Toluene (10.0 vol.) was added to a reaction kettle under nitrogen atmosphere, and then compound 7 (2.67 Kg, 1.0 eq.), compound 8 (1.40 Kg.), potassium carbonate (2.21 Kg, 2.0 eq.), 4,5-bis(diphenylphosphino)-9,9-dimethylxanthene (111.1 g, 0.024 eq.), palladium acetate (35.9 g, 0.02 eq.) and purified water (110.6 g) were added sequentially with stirring. The reaction system was heated to 80±5 °C and maintained at this temperature until the content of the compound 7 in the reaction solution was ≤ 2.0% as detected by HPLC. The reaction system was cooled to 20-30 °C, stirred and centrifuged. The filter cake was washed with toluene (2.0 vol.) and collected, and the filtrate was discarded. A proper amount of dichloromethane and methanol was added to a reaction kettle under nitrogen atmosphere, and the reaction system was stirred. The filter cake was added, and the resulting reaction system was heated to 40±5 °C, stirred, cooled to 20-30 °C and then filtered through celite in a suction filtration tank. The filter cake was washed with a solution of 2% methanol in dichloromethane (V/V), and the filtrate was collected. The filtrate was added to the reaction kettle under nitrogen atmosphere and stirred. Toluene (10.0 vol.) was added, the external temperature was controlled to be not lower than 85 °C, the dichloromethane was distilled out under normal pressure until the internal temperature rose to 75±5 °C, and then the distillation was stopped. The reaction system was cooled to 20-30 °C, stirred and centrifuged. The filter cake was collected, and the filtrate was discarded. The resulting filter cake was dried in a vacuum drying oven to give 2.99 kg of a crude product of the compound 9, and a sample was taken for HPLC analysis. As shown in FIG. 6, the purity is 98.6%. Aqueous hydrochloric acid solution (2 M, 10.0 vol.) was added to the reaction kettle and stirred under nitrogen atmosphere, and then the crude product of the compound 9 (1.0 eq.), dichloromethane (10.0 vol.) and mercapto silica gel (model: PSB-20, wt% = 20%) (526.0 g) were sequentially added. The reaction system was stirred at 30-40 °C and the reaction was stopped until the amount of palladium residue was ≤ 10 ppm as detected by sampling and detection. Suction filtration was performed; the filtrate was separated, and the aqueous phase was taken. Dichloromethane (15.0 vol.) and methanol (1.5 vol.) were added to the aqueous phase, and the pH value was adjusted to 9-11 with aqueous sodium hydroxide solution. Liquid separation was performed, and the organic phase was taken and concentrated under reduced pressure to ≤ 2.0 volumes. The reaction system was cooled to 20-30 °C, stirred for at least 3 h, and then subjected to suction filtration, and the filter cake was collected. The filter cake was dried in an oven, the production was 2.070 Kg, and the yield was 52.4%. A sample was taken for HPLC detection. As shown in FIG. 7, the purity of the compound 9 is 98.97%, and the maximum single impurity is 0.24%.

¹H-NMR (400 MHz, CDCl₃) δ 9.38 (br s, 1H), 8.49 (d, 1H, J=3.2Hz), 8.34-8.33 (m, 2H), 7.96 (s, 1H), 7.88 (d, 1H, *J* =11.2Hz), 7.58 (dd, 1H, J =8.8Hz, 1.6Hz), 3.01-2.98 (m, 2H), 2.52-2.44 (m, 1H), 2.38 (s, 3H), 2.34 (s, 3H), 2.25-2.04 (m, 8H), 1.87-1.76 (m, 6H).

### Example 5: Synthesis of 5-fluoro-4-(7'-fluoro-2'-methylspiro[cyclopentane-1,3'-indol]-5'-yl)-N-(5-(1-methylpiperidin-4-yl)pyridin-2-yl)pyrimidin-2-amine (Compound of Formula (II) or Compound 9)

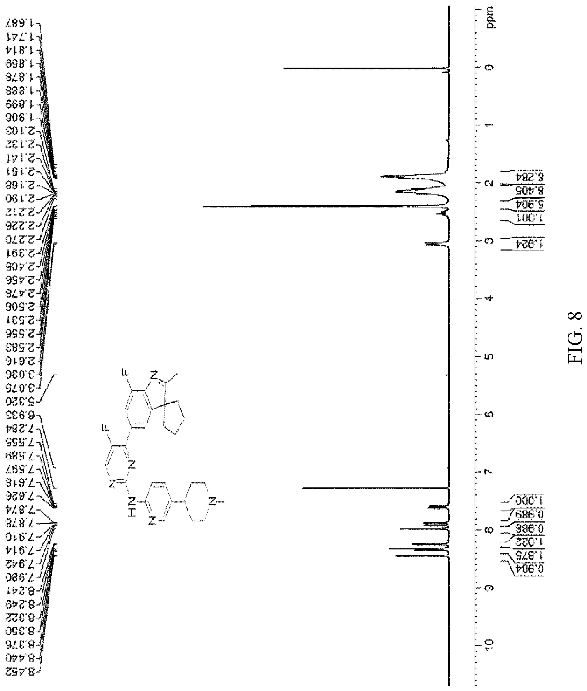

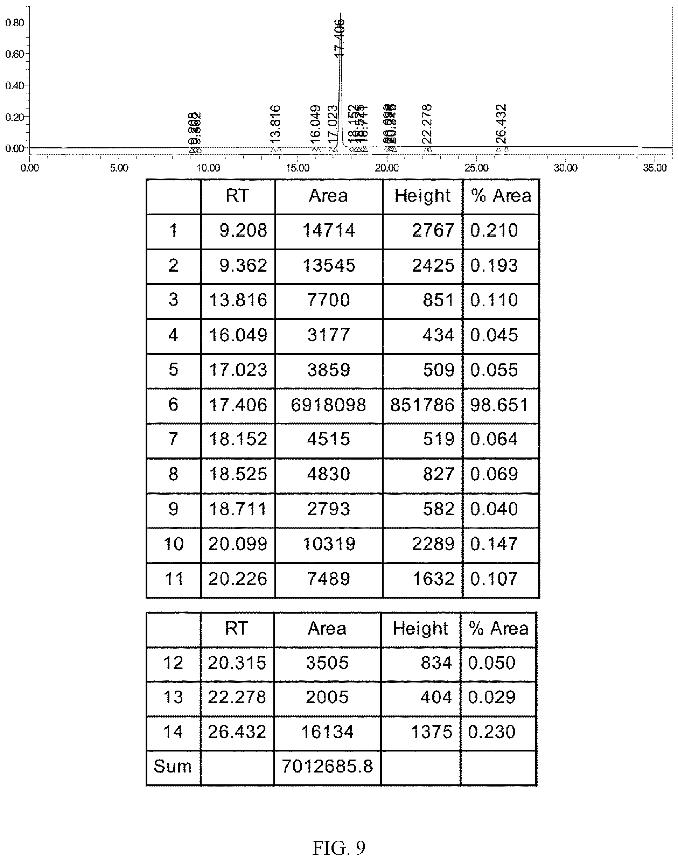

A crude product of the compound 9 was obtained as in Example 4, and then the crude product of the compound 9 was dissolved in hydrochloric acid (2 M, 10 vol.) under nitrogen atmosphere. Then dichloromethane (10 vol.) was added, and the reaction system was heated to 30-40 °C, stirred for 1 h, and filtered through celite. The filter cake was washed twice with purified water (1 vol.), liquid separation was performed, and the aqueous phase was taken. Dichloromethane (10 vol.) was added to the aqueous phase, and the mixture was stirred for 1 h at 30-40 °C and filtered through celite. The filter cake was washed twice with purified water (1 vol.), liquid separation was performed, and the aqueous phase was taken. Dichloromethane (10 vol.) was added to the aqueous phase, and the mixture was stirred for 1 h at 30-40 °C and filtered through celite. The filter cake was washed twice with purified water (1 vol.), liquid separation was performed, and the aqueous phase was taken. Dichloromethane (15.0 vol.) and methanol (1.5 vol.) were added to the aqueous phase, and the pH value was adjusted to 9-11 with aqueous sodium hydroxide solution. Liquid separation was performed, and the organic phase was taken. Mercapto silica gel (model: PSB-20, wt% = 20%) was added, and the mixture was stirred for 16 h at 40 °C and filtered through celite. The organic phase was concentrated under reduced pressure to ≤ 2.0 volumes. The reaction system was cooled to 20-30 °C, stirred for at least 3 h, and then subjected to suction filtration, and the filter cake was collected. The filter cake was dried in an oven, the production was 2.21 Kg, and the yield was 55.9%. A sample was taken for HPLC detection. As shown in FIG. 9, the purity of the compound 9 is 98.65%, and the maximum single impurity is 0.23%. The ¹H-NMR data are shown in FIG. 8.

¹H-NMR (300 MHz, CDCl₃) δ 8.45 (d, 1H), 8.37-7.87 (m, 6H), 3.04 (m, 2H), 2.53 (m, 1H), 2.47 (s, 3H), 2.45 (s, 3H), 2.28-1.69 (m, 16H).

### Example 6: Synthesis of 5-fluoro-4-(7'-fluoro-2'-methylspiro[cyclopentane-1,3'-indol]-5'-yl)-N-(5-(1-methylpiperidin-4-yl)pyridin-2-yl)pyrimidin-2-amine (Compound of Formula (II) or Compound 9)

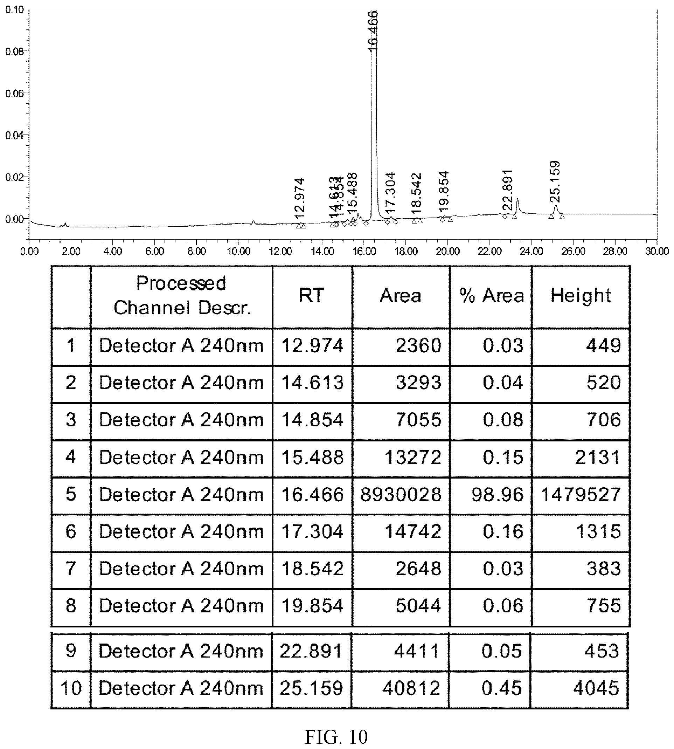

A crude product of the compound 9 was obtained as in Example 4. Aqueous hydrochloric acid solution (2 M, 10.0 vol.) was added to the reaction kettle and stirred under nitrogen atmosphere, and then the crude product of compound 9 (1.0 eq.), dichloromethane (10.0 vol.) and mercapto silica gel (model: PSB-20, wt% = 20%, 526.0 g) were sequentially added. The reaction system was stirred at 30-40 °C and the reaction was stopped until the amount of palladium residue was ≤ 10ppm as detected by sampling and detection. Suction filtration was performed; the filtrate was separated, and the aqueous phase was taken. Dichloromethane (15.0 vol.) and methanol (1.5 vol.) were added to the aqueous phase, and the pH value was adjusted to 9-11 with aqueous sodium hydroxide solution. Liquid separation was performed, and the organic phase was taken and concentrated under reduced pressure to dryness. Toluene (5.0 vol.) was added and distilled to dryness. The mixture was stirred, and toluene (5.0 vol.) and anhydrous ethanol (5.0 vol.) were added. The mixture was heated to reflux until it was clarified, stirred for 15 min while maintaining the temperature, slowly cooled to room temperature, stirred for 5 h, and then subjected to suction filtration. The filter cake was collected and dried in an oven, and its yield was 79.6%. A sample was taken for HPLC analysis. As shown in FIG. 10, the purity of the compound 9 is 98.96%, and the maximum single impurity is 0.45%.

### Example 7: Synthesis of Compound 10

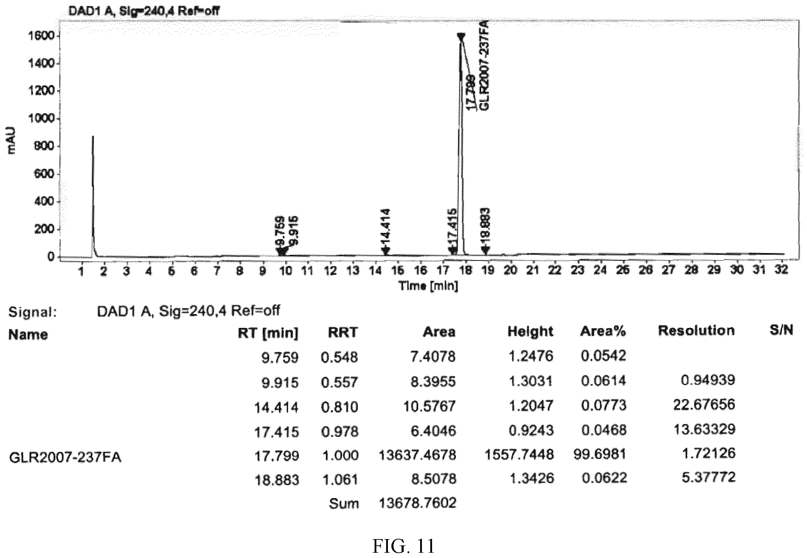

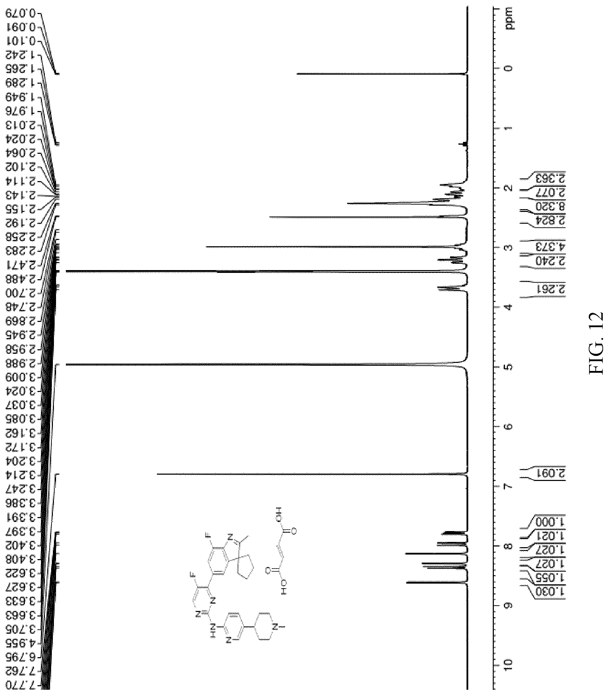

Dichloromethane (22.0 vol.) and ethanol (22.0 vol.) were added to a reaction kettle at room temperature under nitrogen atmosphere, and then the compound 9 (1.0 eq.) was added under mechanical stirring. The reaction system was heated to 30-40 °C and stirred until the solid was almost dissolved to a level of solution clarification. The reaction system was cooled to 20-30 °C, and a solution of the compound 9 in dichloromethane and ethanol was added to another reaction kettle through a microporous filter and stirred. The reaction system was heated, dichloromethane and ethanol were distilled out under normal pressure, and the reaction temperature was maintained at 80±5 °C. A solution of fumaric acid (1.0 eq.) in ethanol (12 vol.) was added dropwise slowly to the reaction kettle through a microporous filter, and then the reaction system was stirred at 80±5 °C overnight. The reaction system was then cooled to 20-30 °C, stirred for at least 1 h and centrifuged. The filter cake was collected, and the filtrate was discarded. The filter cake was dried in a vacuum drying oven. A sample was taken for HPLC analysis. As shown in FIG. 11, the purity of the compound 10 is 99.7%, and the maximum single impurity is 0.077%. The production was 1.605 Kg and the yield was 63.6%. The ¹H-NMR data are shown in FIG. 12, and MS (ESI) m/z = 489.2[M+H]⁺.

### Example 8: Synthesis of Compound 10

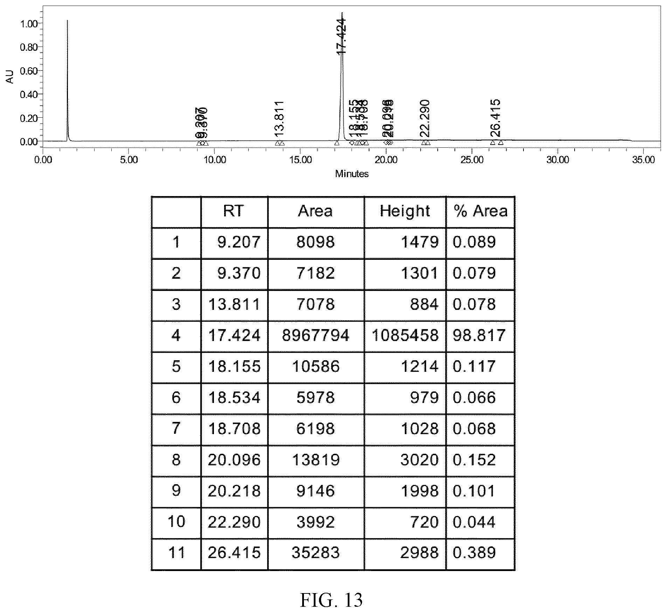

Dichloromethane (10.0 vol.) and ethanol (10.0 vol.) were added to a reaction kettle at room temperature under nitrogen atmosphere, and then the compound 9 (1.0 eq.) was added under mechanical stirring. The reaction system was heated to 30-40 °C and stirred until the solid was almost dissolved to a level of solution clarification. The reaction system was cooled to 20-30 °C, and a solution of the compound 9 in dichloromethane and ethanol was added to another reaction kettle through a microporous filter and stirred. The reaction system was heated, dichloromethane and ethanol were distilled out under normal pressure, and the reaction temperature was maintained at 80±5 °C. A solution of fumaric acid (1.0 eq.) in ethanol (7 vol.) was added dropwise slowly to the reaction kettle through a microporous filter, and then the reaction system was stirred at 80±5 °C overnight. The reaction system was then cooled to 20-30 °C, stirred for at least 1 h and centrifuged. The filter cake was collected, and the filtrate was discarded. The filter cake was dried in a vacuum drying oven. A sample was taken for HPLC analysis. As shown in FIG. 13, the purity of the compound 10 is 98.81%, and the maximum single impurity is 0.39%. The yield was 90%. MS (ESI) m/z = 489.2[M+H]⁺.

### Example 9: Synthesis of Compound 10

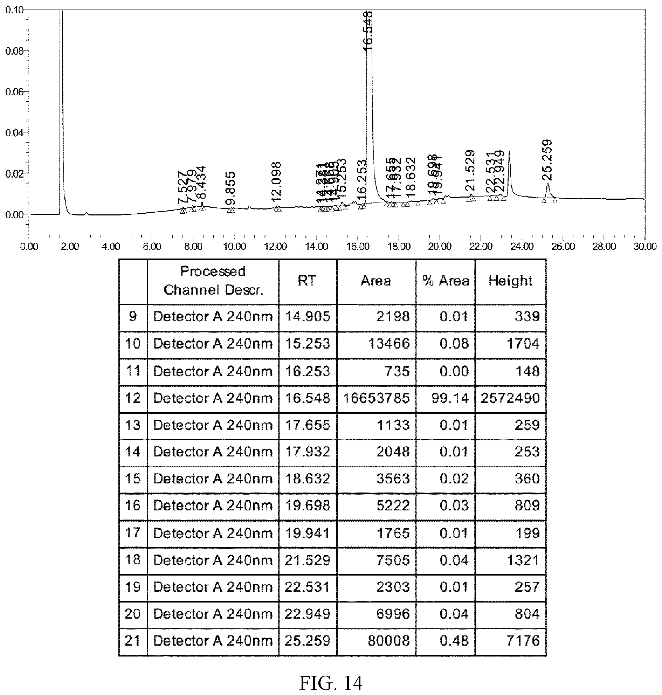

Toluene (10.0 vol.) and ethanol (10.0 vol.) were added to a reaction kettle at room temperature under nitrogen atmosphere, and then the compound 9 (1.0 eq.) was added under mechanical stirring. The reaction system was heated to reflux and stirred until the solid was almost dissolved to a level of solution clarification. The solution was maintained at over 75 °C, added to another reaction kettle through a microporous filter and stirred. The reaction system was heated to 80±5 °C, and a solution of fumaric acid (1.0 eq.) in ethanol (12 vol.) was added dropwise slowly to the reaction kettle, and then the reaction system was stirred at 80±5 °C for 3 h. The reaction system was then slowly cooled to 20-30 °C, stirred for at least 1 h and centrifuged. The filter cake was collected, and the filtrate was discarded. The filter cake was dried in a vacuum drying oven. A sample was taken for HPLC analysis. As shown in FIG. 14, the purity of the compound 10 is 99.14%, the maximum single impurity is 0.48%. The yield was 96.2%. MS (ESI) m/z = 489.2[M+H]⁺.

The present invention has been illustrated by the above examples, but it should be understood that the above examples are for illustrative and descriptive purposes only and are not intended to limit the present invention to the scope of the described examples. Furthermore, it will be understood by those skilled in the art that the present invention is not limited to the examples described above, and that many variations and modifications can be made in accordance with the teachings of the present invention, all of which fall within the scope of the present invention as claimed. The protection scope of the present invention is defined by the appended claims and equivalents thereof.

## Claims

1. A method for preparing a compound of formula (I), comprising:
1) reacting (4-bromo-2-fluorophenyl)hydrazine or a salt thereof with 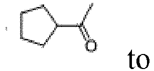 give a compound of formula 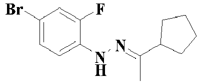 (IV), and
2) forming the compound of formula 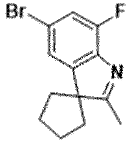 (I) using the compound of formula (IV).

2. The method according to claim 1, wherein the salt of (4-bromo-2-fluorophenyl) hydrazine in the step 1) is 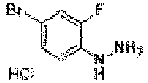 , sulfate of (4-bromo-2-fluorophenyl) hydrazine or *p*-toluenesulfonate of (4-bromo-2-fluorophenyl)hydrazine, preferably

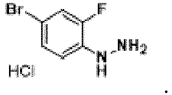

3. The method according to claim 1 or 2, wherein the step 1) is performed under basic or neutral conditions; and/or
the step 2) is performed in the presence of an acid.

4. The method according to claim 3, wherein,
the step 1) is performed in the presence of a base; the base is an inorganic base or an organic base, preferably an organic base, more preferably diisopropylethylamine, triethylamine, pyridine, piperidine or *N*,*N*-dimethylaminopiperidine, still more preferably diisopropylethylamine or triethylamine, and most preferably triethylamine; and/or
the acid is an organic acid, an inorganic acid or a Lewis acid, preferably sulfuric acid, hydrochloric acid, phosphoric acid, methanesulfonic acid, ion exchange resin, *p*-toluenesulfonic acid, acetic acid, ZnCl₂, FeCl₃, AlCl₃ or SnCl₄; and/or
a solvent of the step 1) and the step 2) is an alcohol, preferably a C₁-C₅ linear or branched alkanol, more preferably methanol, ethanol, propanol or isopropanol, and still more preferably methanol or ethanol.

5. The method according to any one of claims 1-4, further comprising: 3) extracting the compound of formula (I) obtained in the step 2) to give a crude product of the compound of formula (I).

6. The method according to claim 5, further comprising: 4) purifying the crude product of the compound of the formula (I) obtained in the step 3) by slurrying.

7. The method according to claim 6, wherein a solvent used for the slurrying is a nonpolar solvent, and the nonpolar solvent is preferably *n*-heptane or *n*-hexane.

8. A method for preparing a compound of formula (V), comprising:
a) preparing a compound of formula (I) by the method according to any one of claims 1-7; and
b) converting the compound of formula (I) to the compound of formula (V):

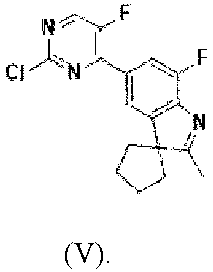

9. The method according to claim 8, wherein the step b) comprises:
(i) converting 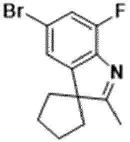 into 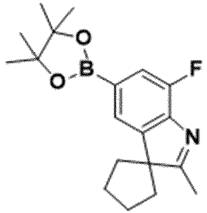 ; and
(ii) converting the 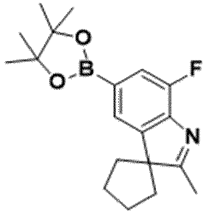 into the compound of formula

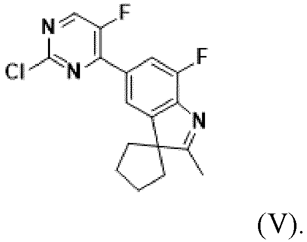

10. The method according to claim 9, wherein the step (ii) further comprises crystallizing the compound of formula (V) after obtaining the compound of formula (V) by reaction.

11. The method according to claim 10, wherein a solvent used for the crystallizing is an alcohol or a ketone;
preferably, the alcohol is a C₁-C₅ linear or branched alkanol; more preferably, the C₁-C₅ linear or branched alkanol is methanol, ethanol, propanol or isopropanol, further preferably methanol or ethanol;
preferably, the ketone is acetone.

12. The method according to claim 10 or 11, further comprising treating the compound of formula (V) with activated carbon before crystallizing the compound of formula (V).

13. The method according to any one of claims 8-12, wherein the step (i) is
performed in the presence of 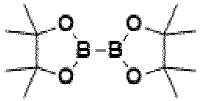 ; and/or
the step (ii) is performed in the presence of 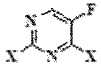 , wherein X in the 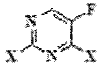 is chlorine, bromine or iodine, preferably chlorine.

14. The method according to claim 13, wherein a reaction solvent of the step (i) is isopropanol or dioxane; and/or a reaction solvent of the step (ii) is toluene or dioxane.

15. A method for preparing 2-amino-5-(1-methylpiperidin)pyridine of formula (VI), comprising:
1) reacting 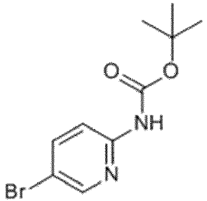 with *N*-methyl-4-piperidone to give 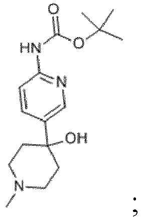
2) converting the 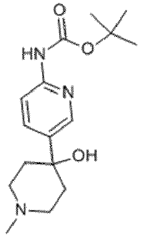 into 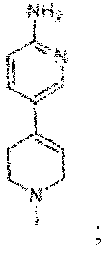 and
3) subjecting the 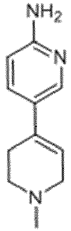 to hydrogenation reaction to give the compound of formula

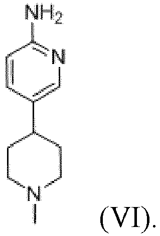

16. The method according to claim 15, wherein, the step 1) is performed in the presence of a Grignard reagent, an organolithium reagent or a combination thereof, preferably in the presence of a Grignard reagent and an organolithium reagent;
preferably, the Grignard reagent is selected from one or more of methylmagnesium chloride, ethylmagnesium chloride, isopropylmagnesium chloride, methylmagnesium bromide, ethylmagnesium bromide and isopropylmagnesium bromide, and more preferably the Grignard reagent is isopropylmagnesium chloride or isopropylmagnesium bromide; and/or
preferably, the organolithium reagent is selected from one or more of *n*-butyllithium, *sec*-butyllithium, *tert*-butyllithium, lithium bis(trimethylsilyl)amide (LiHMDS) and lithium diisopropylamide (LDA), and more preferably, the organolithium reagent is selected from *n*-butyllithium, *sec*-butyllithium and lithium diisopropylamide.

17. The method according to any one of claims 15-16, wherein a crude product of the compound obtained in the step 1) is purified by slurrying.

18. The method according to claim 17, wherein a solvent used for the slurrying is a combination of a nonpolar solvent and a polar solvent;
preferably, the volume ratio of the nonpolar solvent to the polar solvent is 1:1;
preferably, the nonpolar solvent is *n*-heptane or *n*-hexane; and/or
preferably, the polar solvent is methyl tert-butyl ether.

19. The method according to any one of claims 15-18, wherein the step 2) is performed under acidic conditions; preferably, the step 2) is performed in the presence of an acid;
preferably, the acid is selected from one or more of *p*-toluenesulfonic acid, *p-*toluenesulfonic acid monohydrate, trifluoroacetic acid, methanesulfonic acid and hydrochloric acid, and more preferably, the acid is *p*-toluenesulfonic acid or *p-*toluenesulfonic acid monohydrate.

20. The method according to any one of claims 15-19, wherein the pressure of the hydrogenation reaction in the step 3) is 0.1-10 MPa, preferably 0.5-2 MPa, and more preferably 1 MPa; and/or
the step 3) is performed in the presence of a hydrogenation catalyst; preferably, the hydrogenation catalyst is selected from one or more of Pd/C, Pd(OH)₂, Raney nickel, platinum oxide and ammonium formate; preferably, the hydrogenation catalyst is Pd/C or Pd(OH)₂.

21. The method according to any one of claims 15-20, wherein a reaction solvent in the step 3) is an alcohol; preferably, the reaction solvent is selected from one or more of C₁-C₅ linear and branched alkanols; more preferably, the reaction solvent is selected from one or more of methanol, ethanol, propanol and isopropanol; especially preferably, the reaction solvent is ethanol or isopropanol.

22. A method for preparing a compound of formula (II), comprising:
m) preparing a compound of formula (V) by the method according to any one of claims 8-14; and
n) converting the compound of formula (V) into the compound of formula (II)

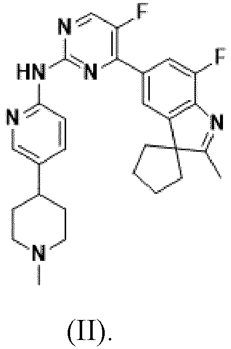

23. The method according to claim 22, wherein the step n) is performed in the presence of a compound of formula (VI) 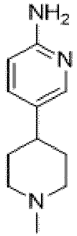 and a palladium catalyst;
preferably, the palladium catalyst is Pd(dppf)Cl₂, Pd(OAc)₂, PdCl₂, Pd(PPh₃)₂Cl₂ or Pd₂(dba)₃, and more preferably, the palladium catalyst is Pd(OAc)₂ or Pd₂(dba)₃.

24. The method according to claim 23, wherein the compound of formula (VI) 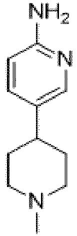 is prepared via the method according to any one of claims 15-21.

25. The method according to any one of claims 22-23, wherein a solvent of the step n) is toluene, benzene or dioxane, preferably toluene.

26. The method according to any one of claims 22-25, wherein the step n) further comprises recrystallizing the compound of formula (II) to obtain a crude product of the compound of formula (II) after obtaining the compound of formula (II) by reaction.

27. The method according to claim 26, wherein solvents used for the recrystallizing comprise a first solvent and a second solvent, wherein the first solvent is toluene or dioxane, and the second solvent is a polar organic solvent; preferably, the second solvent is one or more of an alcohol, a ketone, a halogenated alkane, an ether and an ester; further preferably, the second solvent is one or more of a C₁-C₅ linear or branched alkanol, acetone, dichloromethane, chloroform, tetrahydrofuran and ethyl acetate; more preferably, the second solvent is one or more of methanol, dichloromethane, ethanol, propanol and isopropanol; most preferably, the second solvent is a combination of methanol and dichloromethane.

28. The method according to claim 26 or 27, wherein the step n) further comprises a step of removing palladium from the crude product of the compound of formula (II).

29. The method according to claim 28, wherein the step of removing palladium is performed in the presence of a metal scavenger, preferably in the presence of mercapto silica gel.

30. The method according to any one of claims 28-29, wherein the step n) further comprises a step of filtering resulting reaction solution and extracting resulting filtrate after the step of removing palladium; preferably, the step n) further comprises a step of crystallizing from extract liquid to give the compound of formula (II) or a step of recrystallizing to give the compound of formula (II) after the extracting is completed.

31. The method according to claim 30, wherein the filtrate is extracted with a polar organic solvent; preferably, the polar organic solvent is one or more of an alcohol, a ketone, a halogenated alkane, an ether and an ester; further preferably, the polar organic solvent is one or more of a C₁-C₅ linear or branched alkanol, acetone, dichloromethane, chloroform, tetrahydrofuran and ethyl acetate; more preferably, the polar organic solvent is one or more of methanol, dichloromethane, ethanol, propanol and isopropanol; most preferably, the polar organic solvent is a combination of methanol and dichloromethane; and/or
a solvent used in the step of recrystallizing to give the compound of formula (II) is a combination of toluene and ethanol.

32. Fumarate of 5-fluoro-4-(7'-fluoro-2'-methylspiro[cyclopentane-1,3'-indol]-5'-yl)-*N-*(5-(1-methylpiperidin-4-yl)pyridin-2-yl)pyrimidin-2-amine, having a structure shown as formula (III) below:

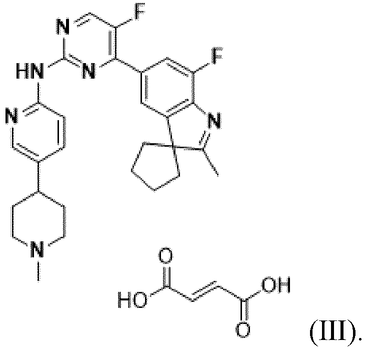

33. A method for preparing fumarate of 5-fluoro-4-(7'-fluoro-2'-methylspiro [cyclopentane-1,3'-indol]-5'-yl)-*N*-(5-(1-methylpiperidin-4-yl)pyridin-2-yl)pyrimidin-2-amine, comprising: reacting a compound of formula (II) with 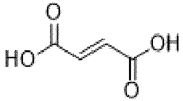 to give a compound of formula (III),

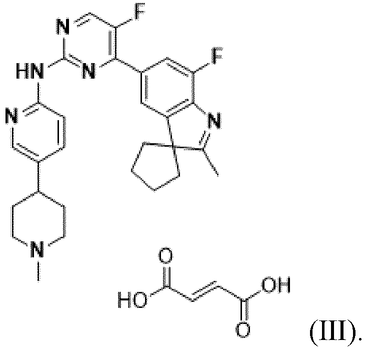

34. The method according to claim 33, wherein the compound of formula (II) is prepared by the method according to any one of claims 22-31.

35. The method according to claim 33 or 34, wherein the reaction is performed in an organic solvent or an aqueous solution of the organic solvent; preferably, the organic solvent is one or more of an alcohol, a ketone, a halogenated alkane, an ether, an ester and an aromatic hydrocarbon; further preferably, the organic solvent is one or more of an alcohol, a ketone, a halogenated alkane, an ether and an ester; more preferably, the organic solvent is one or more of a C₁-C₅ linear or branched alkanol, acetone, ethyl acetate, dichloromethane, chloroform, tetrahydrofuran and a C₁-C₆ linear or branched alkylbenzene; further more preferably, the organic solvent is one or more of a C₁-C₅ linear or branched alkanol, acetone, ethyl acetate, dichloromethane, chloroform and tetrahydrofuran; still more preferably, the organic solvent is one or more of methanol, ethanol, propanol, isopropanol, acetone, dichloromethane, chloroform, toluene and ethylbenzene; even more preferably, the organic solvent is one or more of methanol, ethanol, propanol, isopropanol, acetone, dichloromethane and chloroform; still even more preferably, the reaction solvent is one of ethanol and dichloromethane, a mixture of ethanol and dichloromethane, one of ethanol and toluene, or a mixture of ethanol and toluene.

36. The method according to any one of claims 33-35, further comprising crystallizing the compound of formula (III) after obtaining the compound of formula (III) by reaction; and/or
adding fumaric acid dropwise slowly to the 5-fluoro-4-(7'-fluoro-2'-methylspiro [cyclopentane-1,3'-indol]-5'-yl)-*N*-(5-(1-methylpiperidin-4-yl)pyridin-2-yl)pyrimidin-2-amine while mixing the 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine with the fumaric acid; and/or
reacting the 5-fluoro-4-(7'-fluoro-2'-methylspiro[cyclopentane-1,3'-indol]-5'-yl)-*N-*(5-(1-methylpiperidin-4-yl)pyridin-2-yl)pyrimidin-2-amine with the fumaric acid at a temperature between 20 °C to a temperature of reaction solvent reflux; and/or
dissolving the 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine before reacting the 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine with the fumaric acid, wherein preferably, solvents used for dissolving the 5-fluoro-4-(7'-fluoro-2'-methylspiro [cyclopentane-1,3'-indol]-5'-yl)-*N*-(5-(1-methylpiperidin-4-yl)pyridin-2-yl)pyrimidin-2-amine comprise a third solvent and a fourth solvent, wherein the third solvent is one or more of a ketone, an ether, an ester and an alcohol, preferably one or more alcohols, more preferably one or more of C₁-C₅ linear and branched alkanols, and especially preferably one or more of methanol, ethanol, propanol and isopropanol; the fourth solvent is toluene, dichloromethane, chloroform or any combination thereof; preferably, the fourth solvent is dichloromethane, chloroform or a combination thereof; preferably, the fourth solvent is toluene, dichloromethane or a combination thereof; preferably, the fourth solvent is toluene or dichloromethane; preferably, the mixing volume ratio of the third solvent to the fourth solvent is 1:(0.1-10), preferably 1:(1-3), and most preferably 1:1; and/or
distilling out the solvents used for dissolving the 5-fluoro-4-(7'-fluoro-2'-methylspiro [cyclopentane-1,3'-indol]-5'-yl)-*N*-(5-(1-methylpiperidin-4-yl)pyridin-2-yl)pyrimidin-2-amine before reacting the 5-fluoro-4-(7'-fluoro-2'-methylspiro[cyclopentane-1,3'-indol]-5'-yl)-*N*-(5-(1-methylpiperidin-4-yl)pyridin-2-yl)pyrimidin-2-amine with the fumaric acid.
